# EUROPÄISCHE PATENTANMELDUNG

(11) **EP 1 338 334 A1**
(43) Veröffentlichungstag der Anmeldung: **27.08.2003**
(21) Anmeldenummer: 02003878.2
(22) Anmeldetag: 21.02.2002
(51) Int. Cl.: B01J 13/02, A61K 7/00, A61K 9/50, A61K 9/51

(54) **Mikrokapseln - XVI**

(71) Anmelder: Cognis Iberia, S.L., 08755 Castellbisbal, (Barcelona) (ES)
(72) Erfinder: Viladot, Josep-Luis, Dr., 08018 Barcelona (ES); Caldero, Gabriela, Dr., 08034 Barcelona (ES); De Moragas, Maria, Dr., 08310 Argentona (Barcelona) (ES)
(74) Vertreter: Fabry, Bernd, Dr.

(57) **Zusammenfassung**

Vorgeschlagen werden neue Mikrokapseln mit einem mittleren Durchmesser von 100 nm bis 5 mm, bestehend aus einer Hüllmembran und einer davon umschlossenen, die Wirkstoffe enthaltenden Matrix, welche sich dadurch auszeichnen, dass die Matrix eine kubischen Phase darstellt.

## Beschreibung

### Gebiet der Erfindung

Die Erfindung befindet sich auf dem Gebiet der Kosmetik sowie der Textiltechnik und betrifft neue Mikrokapseln mit darin enthaltenen kubischen Phasen, Verfahren zu deren Herstellung sowie deren Verwendung zur Herstellung von kosmetischen Zubereitungen oder Fasern und Textilien.

### Stand der Technik

Unter dem Begriff "Mikrokapsel" werden vom Fachmann sphärische Aggregate mit einem Durchmesser im Bereich von etwa 0,1 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccaride, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar), *Kuhs Probiol Nanospheres* (Phospholipide) sowie *Primaspheres* und *Primasponges* (Chitosan, Alginate) und *Primasys* (Phospholipide).

In diesem Zusammenhang sei auch auf die deutsche Patentanmeldung **DE 19712978 A1** (Henkel) hingewiesen, aus der Chitosanmikrosphären bekannt sind, die man erhält, indem man Chitosane oder Chitosanderivate mit Ölkörpern vermischt und diese Mischungen in alkalisch eingestellte Tensidlösungen einbringt. Aus der deutschen Patentanmeldung **DE 19756452 A1** (Henkel) ist ferner auch die Verwendung von Chitosan als Verkapselungsmaterial für Tocopherol bekannt. Chitosanmikrokapseln und Verfahren zu ihrer Herstellung sind Gegenstand früherer Patenanmeldungen der Patentanmelderin **[WO 01/01926, WO 01/01927, WO 01/01928, WO 01/01929].**

Den Mikrokapseln des Stands der Technik ist gemeinsam, dass sie die Wirkstoffe unter Zuhilfenahme eines Gelbildners binden, wobei es sich hierbei in der Regel um Heteropolysaccharide, wie beispielsweise Agar Agar oder Proteine, wie Albumin handelt. Von Nachteil ist jedoch, dass diese Gele anisotrop sind, d.h. keine geregelte Struktur aufweisen, so dass die Wirkstoffe nicht homogen verteilt sind. Eine zufällige Verteilung der Wirkstoffe in der Matrix und damit auch in der Kapsel führt jedoch dazu, dass die verzögerte Freisetzung nicht gleichmäßig, sondern mitunter in Schüben erfolgt, abhängig davon, ob gerade eine Region der Kapsel betroffen ist, in der eine lokal hohe oder niedrige Wirkstoffkonzentration vorliegt.

In diesem Zusammenhang sei darauf hingewiesen, dass eine Reihe von Fettstoffen, wie beispielsweise ungesättigte Fettsäurepartialglyceride, innerhalb enger Grenzen mit Wasser hochviskose, flüssig-kristalline Phasen ausbilden können. Diese stellen makroskopisch betrachtet nichts anderes als ein dreidimensionales Tunnelsystem aus hydrophilen und lipophilen Bereichen dar, wobei die Tunnelwände aus Bilayern der Partialglyceride bestehen. Diese Gele werden ihrer Struktur wegen in der Literatur auch kubische Phasen oder kubische Gele genannt und können in Abhängigkeit der Verteilung von hydrophilen und hydrophoben Bereichen "normal" oder "invers" vorliegen [vgl. **La Recherche, Vol. 23, pp 306-315, März 1992; Lipid Technology, Vol.2(2), pp 42-45, April 1990**]. Sie zeichnen sich ferner dadurch aus, dass sie transparent sind und sich im polarisierten Licht als isotrop erweisen. Von besonderem anwendungstechnischen Interesse ist, dass sie in der Lage sind, wasserlösliche, polare Wirkstoffe in sich aufzunehmen, zu speichern und verzögert freizusetzen; dies wird beispielsweise in der internationalen Patentanmeldung **WO 84/02076** sowie der europäischen Patentschrift **EP 0711540 B1** beschrieben.

Die Aufgabe der vorliegenden Erfindung hat folglich darin bestanden, neue Mikrokapseln zur Verfügung zu stellen, die frei von den eingangs geschilderten Nachteilen sind und insbesondere durch eine homogene Verteilung der Wirkstoffe in der Matrix gewährleisten, dass es beim Bruch der Kapsel bzw. durch Diffusion zu einer gleichmäßigen Freisetzung kommt.

### Beschreibung der Erfindung

Gegenstand der Erfindung sind neue Mikrokapseln mit einem mittleren Durchmesser von 100 nm bis 5 mm, bestehend aus einer Hüllmembran und einer davon umschlossenen, die Wirkstoffe enthaltenden Matrix, welche sich dadurch auszeichnen, dass die Matrix eine kubischen Phase darstellt.

Überraschenderweise wurde gefunden, dass sich kubische Phasen sehr leicht verkapseln lassen. Durch den isotropen Aufbau der Gele ist gewährleistet, dass die Wirkstoffe homogen verteilt vorliegen und dementsprechend auch gleichmäßig freigesetzt werden können. Da die Gele eine hohe Viskosität aufweisen zeichnen sich die Kapseln zudem durch eine besondere Härte und Stabilität beispielsweise gegenüber Tensidlösungen und erhöhter Temperatur aus.

### Herstellverfahren I

In einem ersten Verfahren kann die Herstellung der Kapseln erfolgen, indem man in die kubische Phase ein kationisches Polymer, vorzugsweise Chitosan, durch Dispergieren feinverteilt und dann die Dispersion mit einem anionischen Polymer behandelt, welches dann an der Grenzfläche die Membran ausbildet. Dem entsprechend betrifft ein weiterer Gegenstand der Erfindung ein Verfahren zur Herstellung von Mikrokapseln mit einem mittleren Durchmesser von 100 nm bis 5 mm, bestehend aus einer Hüllmembran und einer davon umschlossenen, die Wirkstoffe enthaltenden Matrix, bei dem man
(a) aus ungesättigten Fettsäurepartialglyceriden und Wasser eine kubische Phase herstellt,
(b) die kubische Phase mit Wirkstoffen und Chitosanen belädt,
(c) die so erhaltene Matrix in Wasser oder einer Ölphase dispergiert, und
(d) die so erhaltene Dispersion mit anionischen Polymeren in Kontakt bringt.

Das Verfahren gelingt auch umgekehrt, wenn man also das anionische Polymer in die Matrix einbringt und die Fällung der Kapseln mit dem kationischen Polymer durchführt. Somit betrifft ein weiterer Gegenstand der Erfindung ein Verfahren zur Herstellung von Mikrokapseln mit einem mittleren Durchmesser von 100 nm bis 5 mm, bestehend aus einer Hüllmembran und einer davon umschlossenen, die Wirkstoffe enthaltenden Matrix, bei dem man
(a) aus ungesättigten Fettsäurepartialglyceriden und Wasser eine kubische Phase herstellt,
(b) die kubische Phase mit Wirkstoffen und anionischen Polymeren belädt,
(c) die so erhaltene Matrix in Wasser oder einer Ölphase dispergiert, und
(d) die so erhaltene Dispersion mit einer wässrigen Chitosanlösung in Kontakt bringt.

### Herstellverfahren II

In einem alternativen Herstellverfahren erfolgt die Bildung der Membran durch einen Grenzflächenpolymerisation von zwei Monomeren, die spontan miteinander reagieren. Ein weiterer Gegenstand der Erfindung betrifft somit ein Verfahren zur Herstellung von Mikrokapseln mit einem mittleren Durchmesser von 100 nm bis 5 mm, bestehend aus einer Hüllmembran und einer davon umschlossenen, die Wirkstoffe enthaltenden Matrix, bei dem man
(a) aus ungesättigten Fettsäurepartialglyceriden und Wasser eine kubische Phase herstellt,
(b) die kubische Phase mit Wirkstoffen und einem Alkylendiamin belädt,
(c) die so erhaltene Matrix in einer Ölphase dispergiert, und
(d) die so erhaltene Dispersion mit einem Dicarbonsäurechlorid in Kontakt bringt.

Auch hier kann das Verfahren wiederum umgekehrt erfolgen, wobei man jedoch bei der Wahl des Dispergiermittels die Polarität des Monomers berücksichtigen muss. Somit betrifft ein weiterer Gegenstand der Erfindung ein Verfahren zur Herstellung von Mikrokapseln mit einem mittleren Durchmesser von 100 nm bis 5 mm, bestehend aus einer Hüllmembran und einer davon umschlossenen, die Wirkstoffe enthaltenden Matrix, bei dem man
(a) aus ungesättigten Fettsäurepartialglyceriden und Wasser eine kubische Phase herstellt,
(b) die kubische Phase mit Wirkstoffen und einem Dicarbonsäurechlorid belädt,
(c) die so erhaltene Matrix in Wasser dispergiert, und
(d) die so erhaltene Dispersion mit einem Alkylendiamin in Kontakt bringt.

### Herstellverfahren III

Ein drittes alternatives Verfahren macht sich den Effekt zu Nutze, dass bestimmte Stoffe bei Änderung der Temperatur oder des pH-Wertes ihre Struktur verändern. Dies ist beispielsweise bei Proteinen der Fall, die bekanntlich oberhalb einer bestimmten Temperatur denaturieren und feste Aggregate bilden. Ein weiterer Gegenstand der Erfindung betrifft daher ein Verfahren zur Herstellung von Mikrokapseln mit einem mittleren Durchmesser von 100 nm bis 5 mm, bestehend aus einer Hüllmembran und einer davon umschlossenen, die Wirkstoffe enthaltenden Matrix, bei dem man
(a) aus ungesättigten Fettsäurepartialglyceriden und Wasser eine kubische Phase herstellt,
(b) die kubische Phase mit Wirkstoffen und Proteinen belädt, und
(c) die so erhaltene proteinhaltige Matrix durch Temperaturerhöhung denaturiert.

### Kubische Phasen

Auf die Natur der kubischen Phasen ist bereits oben eingegangen worden. Als Ausgangsstoffe eignen sich ungesättigte Fettsäurepartialglyceride, wie beispielsweise die Monound/oder Diglyceride der Palmoleinsäure, Ölsäure, Elaidinsäure, Petroselinsäure, Linolsäure, konjugierten Linolsäure, Linolensäure, Ricinolsäure, Gadoleinsäure, Behensäure und Clupanodonsäure. Vorzugsweise werden Ölsäuremono/diglyceride eingesetzt, die beispielsweise unter der Bezeichnung Monomuls 90 0-18 (Cognis Deutschland GmbH & Co. KG) im Handel sind. In der Regel setzt man ungesättigte Fettsäurepartialglyceride und Wasser im Gewichtsverhältnis 50 : 50 bis 90 : 10, vorzugsweise 60 : 40 bis 70 : 30 ein. Die Herstellung der kubischen Phasen kann in an sich bekannter Weise erfolgen, also durch Vermischen der Einsatzstoffe unter starker Scherung. Bezüglich der Herstellung und der Beladung der Phasen mit Wirkstoffen sei auf die Lehre der schon eingangs zitierten **EP 0711540 B1** verwiesen. Bei der Herstellung der kubischen Phasen können alternativ auch wässrige Lösungen eingesetzt werden, die bereits wasserlösliche Monomere, Polymere oder Wirkstoffe enthalten.

### Wirkstoffe für kosmetische und pharmazeutische Anwendungen

Die Auswahl der Wirkstoffe ist insofern unkritisch, als dass es ihrer Natur nach praktisch keine Limitierungen gibt. Da es sich bei den kubischen Phasen jedoch um wässrige Systeme handelt werden vorzugsweise wasserlösliche bzw. wasserdispergierbare Wirkstoffe eingesetzt, da diese sich leichter und homogener in der kubischen Phasen verteilen lassen. Typische Beispiele für geeignete Wirkstoffe sind
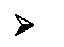 Vitamine (z.B. Tocopherole, Tocopherolacetat, Tocopherolpalmitat, Carotinoide, Ascorbinsäure),
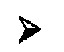 Enzyme,
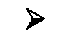 Pflanzenextrakte (z.B. Extrakte des Ginkgo bilabo, Vaccinium myrtillus, Vinis vitalis, Thea vinensis, Triflolium pratense, Prunus dulcis, Waltheria indica),
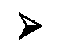 Oligosaccharide (z.B. Glucane),
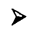 Biopolymere (z.B. Chitosan, Cyclodextrin),
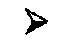 Proteine,
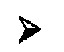 Nucleinsäuren (Ribonucleinsäuren, Desoxyribonucleinsäuren und deren Fragmentierungsprodukte) sowie
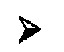 Bioactive Reagenzien (z.B. Dihydroxyaceton, Arbutin, Ferulasäure, Kojisäure, Cumarinsäure, Menthol, Coffein, Retinol, Bisabolol, Allantoin, Panthenol, Phytantriol, AHA-Säuren, Hyaluronsäure, Ceramide, Pseudoceramide, Flavonoide, Isoflavonoide, Isoflavonglucoside, Polyphenole, Astaxanthin, Squalan, Squalen).

Die Mikrokapseln, welche die in den kubischen Phasen dispergierten Wirkstoffe enthalten, werden in der Regel topisch zur Anwendung gebracht. In einzelnen Fällen kommt jedoch auch eine orale Aufnahme in Frage.

### Wirkstoffe für die textile Ausrüstung

Die oben genannten mit kosmetischen Wirkstoffen beladenen Mikrokapseln können auch zur Ausrüstung von Textilien dienen. Zu diesem Zweck werden die Fasern oder textilen Flächegebilden entweder mit Suspensionen der Mikrokapseln imprägniert und dann getrocknet oder aber es erfolgt eine Zwangsapplikation nach dem Fullerverfahren. Die so ausgerüsteten Stoffe, beispielsweise Unterwäsche oder Strümpfe bzw. Strumpfhosen setzen während des Tragens die kosmetischen Wirkstoffe frei und gewährleisten so einen verbesserten Tragekomfort.

Ein weiterer Einsatzbereich der neuen Mikrokapseln besteht in der Ausrüstung von Textilien mit Flammschutzmitteln. Der besondere Vorteil liegt hier darin, dass die Kapseln bei deutlich höheren Temperaturen erweichen und das Flammschutzmittel nicht zu früh freisetzen. Typische Beispiele für geeignete Wirkstoffe sind alle Arten von Flammschutzmitteln, also Metalloxide, wie z.B. Magnesiumoxid, Calciumoxid, Aluminiumoxid oder Antimon(III)oxid, organische Halide, wie z.B. Tetrabrombisphenol A oder Decabromodiphenyloxid sowie insbesondere organische Phosphorverbindungen, wie z.B. Triphenylphosphat (TPP), Tricresylphosphat (TCP), Cresyldiphenylphosphat (CDP) oder Tetraphenyldiphosphat. Die Applikation erfolgt auch hier entweder durch ein Imprägnierbad oder durch Füllern.

Üblicherweise setzt man die kosmetischen Wirkstoffe bzw. die Flammschutzmittel - bezogen auf die kubische Phase - in Mengen von 1 bis 25, vorzugsweise 2 bis 20 und insbesondere 5 bis 10 Gew.-% ein.

### Herstellung der Membran durch Fällung

Wie schon eingangs erwähnt, kann die Herstellung der Mikrokapseln nach einem ersten Verfahren durch eine Fällungsreaktion zwischen einem anionischen und einem kationischen Polymer erfolgen, wobei das eine Polymer in der kubischen Phase dispergiert vorliegt, die dann mit dem entgegengesetzt geladenen Polymer in Kontakt gebracht wird.

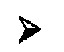 Kationische Polymere

Als kationische Polymere haben sich Chitosane bewährt, da diese eine besonders hohe dermatologische und ökotoxikologische Verträglichkeit aufweisen und besonders rasch sehr stabile Membranen ausbilden. Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

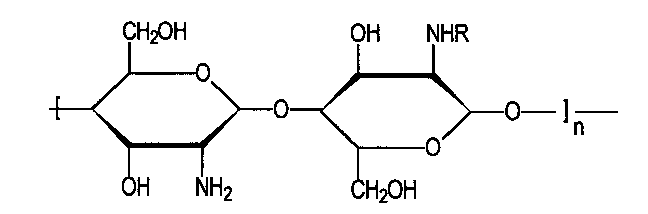

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's En** **cyclopedia of Industrial Chemistry, 5th Ed., Vol. A6, Weinheim, Verlag Chemie, 1986**, **S. 231-232)**. Übersichten zu diesem Thema sind auch beispielsweise von B. Gesslein et al. in **HAPPI 27, 57 (1990),** O. Skaugrud in **Drug Cosm.Ind. 148, 24 (1991)** und E. Onsoyen et al. in **Seifen-Öle-Fette-Wachse 117, 633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol. Chem. 177, 3589 (1976)** oder der französischen Patentanmeldung **FR 2701266 A** bekannt. Vorzugsweise werden solche Typen eingesetzt, wie sie in den deutschen Patentanmeldungen **DE 4442987 A1** und **DE 19537001 A1** (Henkel) offenbart werden und die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

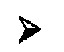 Anionische Polymere

Als anionische Polymere, die mit den Kationpolymeren durch Fällung Membranen ausbilden, kommen vorzugsweise die Salze der Alginsäure, anionische Chitosanderivate, Poly(meth)acrylate und Carboxymethylcellulosen in Frage. Bei der Alginsäure handelt es sich um ein Gemisch carboxylgruppenhaltiger Polysaccharide mit folgendem idealisierten Monomerbaustein:

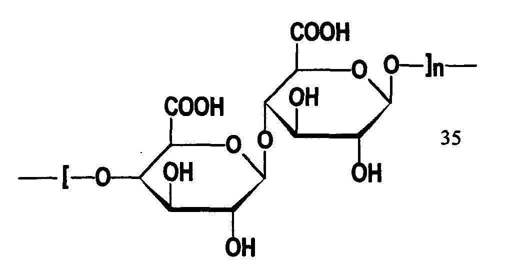

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage, wie sie beispielsweise in der deutschen Patentschrift **DE 3713099 C2** (L'Oréal) sowie der deutschen Patentanmeldung **DE 19604180 A1** (Henkel) beschrieben werden. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.

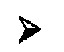 Ölphase

Zur Herstellung von besonders feinteiligen Mikrokapseln hat es sich als vorteilhaft erwiesen, die Matrices vor der Ausbildung der Membran in einer Ölphase zu dispergieren, die später wieder abgetrennt und zurückgewonnen wird. Als Öle kommen für diesen Zweck beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

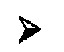 Herstellung der Matrix und Fällung der Membran

Zur Herstellung der Mikrokapseln wird das kubische Gel vorgelegt und unter starker Scherung, gegebenenfalls unter Erwärmung mit einer wässrigen Lösung versetzt, welche die Chitosane bzw. die Anionpolymere in Mengen von 5 bis 25 Gew.-% sowie die Wirkstoffe in Mengen von 1 bis 5 Gew.-% - jeweils bezogen auf die kubischen Phase - enthalten; diese Mischung wird als Matrix bezeichnet. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus kubischer Phase, Polymer und Wirkstoffen kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des in der Matrix enthaltenen Polymers mit den entgegengesetzt geladenen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Polymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 0,01 bis 5 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig.

### Herstellung der Membran durch Polymerisation

In ähnlicher Weise wie oben beschrieben kann die Bildung der Membran auch durch eine Polymerisation von zwei Monomeren an der Phasengrenze erfolgen. Vorzugsweise werden die Monomere so ausgewählt, dass sie spontan, quantitativ und ohne Zusatz von Aktivatoren abreagieren. Dies ist beispielsweise bei der Reaktion von Alkylendiaminen mit Dicarbonsärechloriden der Fall.

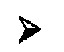 Alkylendiamine

Geeignete Alkylendiamine folgen vorzugsweise der Formel **(I),**

**H**_{**2**}**N(CH**_{**2**}**)**_{**n**}**NH**_{**2**} (I)

in der n für Zahlen von 4 bis 10, vorzugsweise 6 bis 8 steht. Bevorzugtes Beispiel ist Hexamethylendiamin.

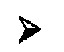 Dicarbonsäurechloride

Dicarbonsäurechloride, die als gegensätzliche Monomere in Frage kommen, folgen vorzugsweise der Formel **(II),**

**ClOC(CH**_{**2**}**)**_{**m**}**COCl** (II)

in der m für Zahlen von 2 bis 22, vorzugsweise 6 bis 16 steht. Typische Beispiele sind Adipinsäurechlorid, Sebacinsäurechlorid sowie das Chlorid der 1,18-Octadecandicarbonsäure, welche durch Biooxidation von Kohlenwasserstoffen entsprechender Kettenlänge zugänglich ist.

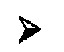 Herstellung der Matrix und Polymerisation der Membran

Zur Herstellung der Mikrokapseln wird das kubische Gel wie oben beschrieben vorgelegt und unter starker Scherung, gegebenenfalls unter Erwärmung mit einer wässrigen Lösung versetzt, welche die Dicarbonsäurechloride in Mengen von 5 bis 25 Gew.-% sowie die Wirkstoffe in Mengen von 1 bis 5 Gew.-% - jeweils bezogen auf die kubischen Phase - enthalten; diese Mischung wird als Matrix bezeichnet. Falls gewünscht, können zu diesem Zeitpunkt zur Viskositätseinstellung wiederum wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Alternativ können anstelle der Dicarbonsäurechloride auch die Alkylendiamine in der Matrix enthalten sein. Wegen ihres unpolaren Charakters empfiehlt es sich dann jedoch, die Matrix nicht in Wasser, sondern in einer Ölphase zu dispergieren. Anschließend erfolgt dann die eigentliche Verkapselung, d.h. die Ausbildung der Hüllmembran durch Inkontaktbringen des in der Matrix enthaltenen ersten Monomers mit den entgegengesetzt geladenen zweiten Monomer. Hierzu empfiehlt es sich, die in der Wasser- oder Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige Lösung des zweiten Monomers zu behandeln. Die dabei resultierenden Zubereitungen weisen in der Regel einen Mikrokapselgehalt im Bereich von 1 bis 10 Gew.-% auf. Nach Filtration werden Mikrokapseln erhalten, welche im Mittel einen Durchmesser im Bereich von vorzugsweise etwa 0,01 bis 5 mm aufweisen. Es empfiehlt sich, die Kapseln zu sieben, um eine möglichst gleichmäßige Größenverteilung sicherzustellen. Auch die auf diesem alternativen Weg erhaltenen Mikrokapseln können im herstellungsbedingten Rahmen eine beliebige Form aufweisen, sie sind jedoch bevorzugt näherungsweise kugelförmig.

### Herstellung der Mikrokapseln durch Denaturierung

Schließlich kann die Verkapselung auch dadurch herbeigeführt werden, dass ein in der Matrix enthaltenes Protein beispielsweise durch Temperaturerhöhung seine Struktur ändert. Als Proteine werden für diesen Zweck insbesondere Albumine eingesetzt, die bei 35 bis 70 °C denaturiert werden und dabei unter dem Einfluss starker Scherung sphärische Gebilde formen, die die Matrix und die darin dispergierten Wirkstoffe einschließt bzw. zusammen hält.

### Gewerbliche Anwendbarkeit

Weitere Gegenstände der Erfindung betreffen die Verwendung der neuen Mikrokapseln zur Herstellung von kosmetischen und/oder pharmazeutischen Zubereitungen sowie zur Ausrüstung von Fasern und textilen Flächengebilden, wobei sie in Mengen von jeweils 0,1 bis 10 und insbesondere 1 bis 5 Gew.-% zum Einsatz gelangen können.

### Kosmetische und/oder pharmazeutische Zubereitungen

Bei den kosmetischen oder pharmazeutischen Zubereitungen, welche die erfindungsgemäßen Mikrokapseln enthalten können, handelt es sich üblicherweise um Cremes, Gele, Lotionen, alkoholische und wässrig/alkoholische Lösungen, Emulsionen, Wachs/Fett-Massen, Stiftpräparate, Puder oder Salben. Sie können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Antioxidantien, Deodorantien, Antitranspirantien, Antischuppenmittel, Filmbildner, Quellmittel, Insektenrepellentien, Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, A-midseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, A-cylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1**), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
> Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
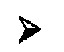 Alkyl- und/oder Alkenyloligoglykoside mit 8 bis 22 Kohlenstoffatomen im Alk(en)ylrest und deren ethoxylierte Analoga;
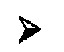 Anlagerungsprodukte von 1 bis 15 Mol Ethylenoxid an Ricinusöl und/oder gehärtetes Ricinusöl;
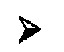 Anlagerungsprodukte von 15 bis 60 Mol Ethylenoxid an Ricinusöl und/oder gehärtetes Ricinusöl;
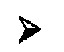 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
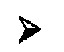 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
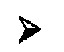 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
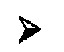 Mono-, Di- und Trialkylphosphate sowie Mono-, Di- und/oder Tri-PEG-alkylphosphate und deren Salze;
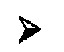 Wollwachsalkohole;
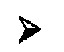 Polysiloxan-Polyalkyl-Polyether-Copolymere bzw. entsprechende Derivate;
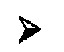 Block-Copolymere z.B. Polyethylenglycol-30 Dipolyhydroxystearate;
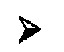 Polymeremulgatoren, z.B. Pemulen-Typen (TR-1,TR-2) von Goodrich;
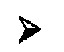 Polyalkylenglycole sowie
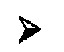 Glycerincarbonat.

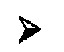 Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

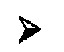 Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen, Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

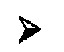 Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

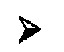 Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

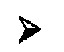 Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

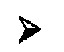 Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

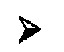 Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylatund eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminiumund/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toil. 108, 95 (1993)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil**. **91, 27 (1976).**

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
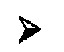 3-Benzylidencampher bzw. 3-Benzylidennorcampher und dessen Derivate, z.B. 3-(4-Methylbenzyliden)campher wie in der **EP 0693471 B1** beschrieben;
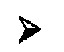 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
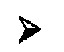 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
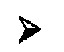 Ester der Salicylsäure, vorzugsweise Salicylsäure-2-ethylhexylester, Salicylsäure-4-isopropylbenzylester, Salicylsäurehomomenthylester;
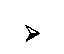 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
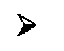 Ester der Benzalmalonsäure, vorzugsweise 4-Methoxybenzmalonsäuredi-2-ethylhexylester;
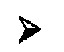 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
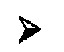 Propan-1,3-dione, wie z.B. 1-(4-tert.Butylphenyl)-3-(4'methoxyphenyl)propan-1,3-dion;
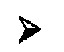 Ketotricyclo(5.2.1.0)decan-Derivate, wie in der **EP 0694521 B1** beschrieben.

Als wasserlösliche Substanzen kommen in Frage:
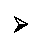 2-Phenylbenzimidazol-5-sulfonsäure und deren Alkali-, Erdalkali-, Ammonium-, Alkylammonium-, Alkanolammonium- und Glucammoniumsalze;
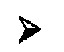 Sulfonsäurederivate von Benzophenonen, vorzugsweise 2-Hydroxy-4-methoxybenzophenon-5-sulfonsäure und ihre Salze;
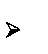 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethylhexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** sowie **Parf.Kosm. 3, 11 (1999)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

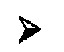 Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

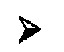 Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

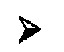 Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

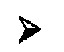 Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
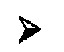 adstringierende Wirkstoffe,
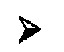 Ölkomponenten,
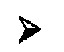 nichtionische Emulgatoren,
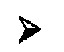 Coemulgatoren,
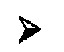 Konsistenzgeber,
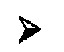 Hilfsstoffe wie z. B. Verdicker oder Komplexierungsmittel und/oder
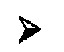 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkoniumtetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
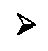 entzündungshemmende, hautschützende oder wohlriechende ätherische Öle,
s 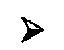 ynthetische hautschützende Wirkstoffe und/oder
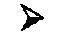 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
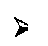 Glycerin;
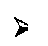 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
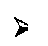 technische Oligoglyceringemische mit einem Eigenkondensationsgrad von 1,5 bis 10 wie etwa technische Diglyceringemische mit einem Diglyceringehalt von 40 bis 50 Gew.-%;
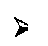 Methyolverbindungen, wie insbesondere Trimethylolethan, Trimethylolpropan, Trimethylolbutan, Pentaerythrit und Dipentaerythrit;
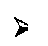 Niedrigalkylglucoside, insbesondere solche mit 1 bis 8 Kohlenstoffen im Alkylrest, wie beispielsweise Methyl- und Butylglucosid;
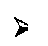 Zuckeralkohole mit 5 bis 12 Kohlenstoffatomen, wie beispielsweise Sorbit oder Mannit,
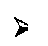 Zucker mit 5 bis 12 Kohlenstoffatomen, wie beispielsweise Glucose oder Saccharose;
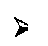 Aminozucker, wie beispielsweise Glucamin;
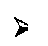 Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Y-lang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

**Beispiel 1.** 50 g einer kubischen Phase bestehend aus 70 Gew.-% Öisäuremono/digiycerid (Monomuls® 90 0-18, Cognis Deutschland GmbH & Co. KG) und 30 Gew.-% Wasser wurden mit 2 g Retinol beladen und dann unter starkem Rühren in 250 ml einer 5 Gew.-%igen wässrigen Natriumalginatlösung dispergiert. Anschließend wurde die resultierende Matrix unter Rühren portionsweise mit 500 ml einer 2 Gew.-%igen Lösung von Chitosan in Glycolsäure (Hydagen® HCMF, Cognis) versetzt. Die resultierenden Mikrokapseln wurden von der wässrigen Phase abfiltriert, mit verdünnter Natriumlaurylsulfatlösung gewaschen, getrocknet und gesiebt. Sie wiesen einen mittleren Durchmesser von 0,5 mm auf.

**Beispiel 2.** 50 g einer kubischen Phase bestehend aus 70 Gew.-% Ölsäuremono/digiycerid (Monomuls® 90 0-18, Cognis Deutschland GmbH & Co. KG) und 30 Gew.-% Wasser wurden mit 2 g Ascorbinsäure beladen und dann unter starkem Rühren in 250 ml einer 5 Gew.-%igen wässrigen Natriumalginatlösung dispergiert. Anschließend wurde die resultierende Matrix unter Rühren portionsweise mit 500 ml einer 2 Gew.-%igen Lösung von Chitosan in Glycolsäure (Hydagen® HCMF, Cognis) versetzt. Die resultierenden Mikrokapseln wurden von der wässrigen Phase abfiltriert, mit verdünnter Natriumlaurylsulfatlösung gewaschen, getrocknet und gesiebt. Sie wiesen einen mittleren Durchmesser von 0,5 mm auf.

**Beispiel 3.** 50 g einer kubischen Phase bestehend aus 70 Gew.-% Ölsäuremono/digiycerid (Monomuls® 90 0-18, Cognis Deutschland GmbH & Co. KG) und 30 Gew.-% einer wässrigen, 10 Gew.-%igen Lösung von Carboxymethylcellulose wurden zusammen mit 1,5 g Desoxyribonucleinsäure unter starkem Rühren in 250 ml Paraffinöl dispergiert. Anschließend wurde die resultierende Matrix mit 500 ml einer 2 Gew.-%igen Lösung von Chitosan in Glycolsäure (Hydagen® HCMF, Cognis) versetzt. Die resultierenden Mikrokapseln wurden von der Ölphase abfiltriert, mit verdünnter Natriumlaurylsulfatlösung gewaschen, getrocknet und gesiebt. Sie wiesen einen mittleren Durchmesser von 0,1 mm auf.

**Beispiel 4.** 50 g einer kubischen Phase bestehend aus 70 Gew.-% Ölsäuremono/digiycerid (Monomuls® 90 0-18, Cognis Deutschland GmbH & Co. KG) und 30 Gew.-% Wasser wurden mit 2 g Squalan beladen und dann unter starkem Rühren in 250 ml einer 2 Gew.-%igen Lösung von Chitosan in Glycolsäure (Hydagen® HCMF, Cognis dispergiert. Anschließend wurde die resultierende Matrix portionsweise mit 250 ml einer 5 Gew.-%igen wässrigen Natriumalginatlösung versetzt. Die resultierenden Mikrokapseln wurden von der wässrigen Phase abfiltriert, mit verdünnter Natriumlaurylsulfatlösung gewaschen, getrocknet und gesiebt. Sie wiesen einen mittleren Durchmesser von 0,5 mm auf.

**Beispiel 5.** 50 g einer kubischen Phase bestehend aus 70 Gew.-% Ölsäuremono/diglycerid (Monomuls® 90 0-18, Cognis Deutschland GmbH & Co. KG) und 30 Gew.-% Wasser wurden zusammen mit 2 g Squalan und 10 g Hexamethylendiamin unter starkem Rühren in 250 ml Mineralöl. Anschließend wurde die resultierende Matrix unter Rühren mit 10 g Sebacinsäurechlorid versetzt. Die resultierenden Mikrokapseln wurden von der Ölphase abfiltriert, mit verdünnter Natriumlaurylsulfatlösung gewaschen, getrocknet und gesiebt. Sie wiesen einen mittleren Durchmesser von 0,1 mm auf.

**Beispiel 6.** 50 g einer kubischen Phase bestehend aus 70 Gew.-% Ölsäuremono/diglycerid (Monomuls® 90 0-18, Cognis Deutschland GmbH & Co. KG) und 30 Gew.-% Wasser wurden zusammen mit 2 g Tocopherol und 10 g Hexamethylendiamin unter starkem Rühren in 250 ml Mineralöl. Anschließend wurde die resultierende Matrix unter Rühren mit 10 g 1,18-Octadecandicarbonsäurechlorid versetzt. Die resultierenden Mikrokapseln wurden von der Ölphase abfiltriert, mit verdünnter Natriumlaurylsulfatlösung gewaschen, getrocknet und gesiebt. Sie wiesen einen mittleren Durchmesser von 0,1 mm auf.

**Beispiel 7**. 50 g einer kubischen Phase bestehend aus 70 Gew.-% Öisäuremono/diglycerid (Monomuls® 90 0-18, Cognis Deutschland GmbH & Co. KG) und 30 Gew.-% Wasser wurden mit 2 g Menthol beladen und unter starkem Rühren in 250 ml einer 5 Gew.-%igen Lösung von Sebacinsäurechlorid dispergiert. Anschließend wurde die resultierende Matrix unter Rühren mit 10 g Hexamethylendiamin versetzt. Die resultierenden Mikrokapseln wurden von der wässrigen Phase abfiltriert, mit verdünnter Natriumlaurylsulfatlösung gewaschen, getrocknet und gesiebt. Sie wiesen einen mittleren Durchmesser von 0,1 mm auf.

**Beispiel 8.** 50 g einer kubischen Phase bestehend aus 70 Gew.-% Öisäuremono/digiycerid (Monomuls® 90 0-18, Cognis Deutschland GmbH & Co. KG) und 30 Gew.-% Wasser wurden mit 2 g Kojisäure beladen und unter starkem Rühren in 250 ml einer 5 Gew.-%igen Lösung von Adipinsäurechlorid dispergiert. Anschließend wurde die resultierende Matrix unter Rühren mit 10 g Hexamethylendiamin versetzt. Die resultierenden Mikrokapseln wurden von der wässrigen Phase abfiltriert, mit verdünnter Natriumlaurylsulfatlösung gewaschen, getrocknet und gesiebt. Sie wiesen einen mittleren Durchmesser von 0,1 mm auf.

**Beispiel 9.** 50 g einer kubischen Phase bestehend aus 70 Gew.-% Öisäuremono/digiycerid (Monomuls® 90 0-18, Cognis Deutschland GmbH & Co. KG) und 30 Gew.-% Wasser wurden unter starkem Rühren mit 2 g Menthol und 20 g Albumin in 200 ml Wasser dispergiert. Anschließend wurde die Mischung über einen Zeitraum von 10 min auf 70 °C erhitzt. Die resultierenden Mikrokapseln wurden von der wässrigen Phase abfiltriert, mit verdünnter Natriumlaurylsulfatlösung gewaschen, getrocknet und gesiebt. Sie wiesen einen mittleren Durchmesser von 0,8 mm auf.

**Beispiel 10.** 50 g einer kubischen Phase bestehend aus 70 Gew.-% Ölsäuremono/diglycerid (Monomuls® 90 0-18, Cognis Deutschland GmbH & Co. KG) und 30 Gew.-% Wasser wurden unter starkem Rühren mit 2 g Triphenylphosphat und 20 g Albumin in 200 ml Wasser dispergiert. Anschließend wurde die Mischung über einen Zeitraum von 10 min auf 70 °C erhitzt. Die resultierenden Mikrokapseln wurden von der wässrigen Phase abfiltriert, mit verdünnter Natriumlaurylsulfatlösung gewaschen, getrocknet und gesiebt. Sie wiesen einen mittleren Durchmesser von 0,8 mm auf.

In Tabelle 1 sind eine Reihe von Beispielen enthalten.

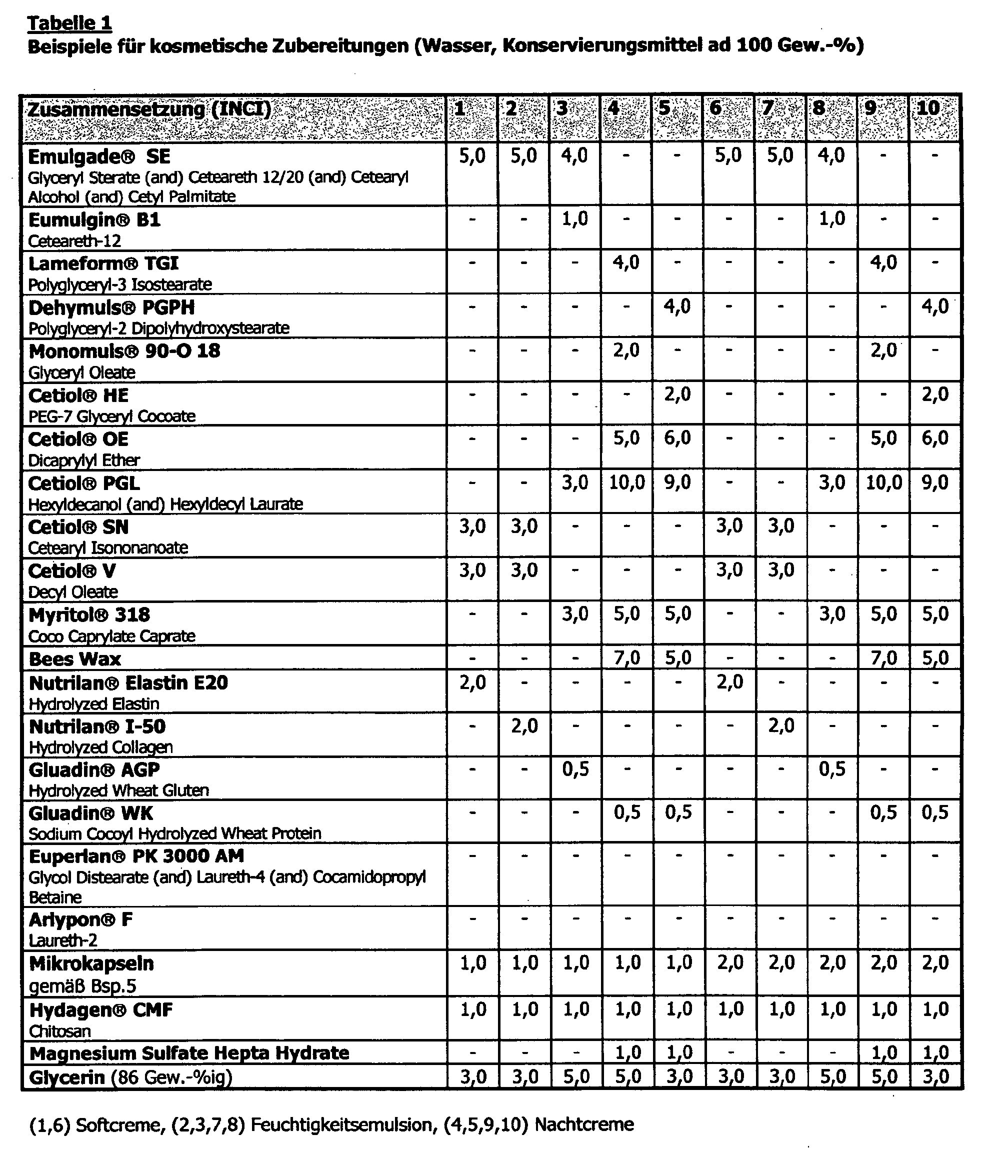

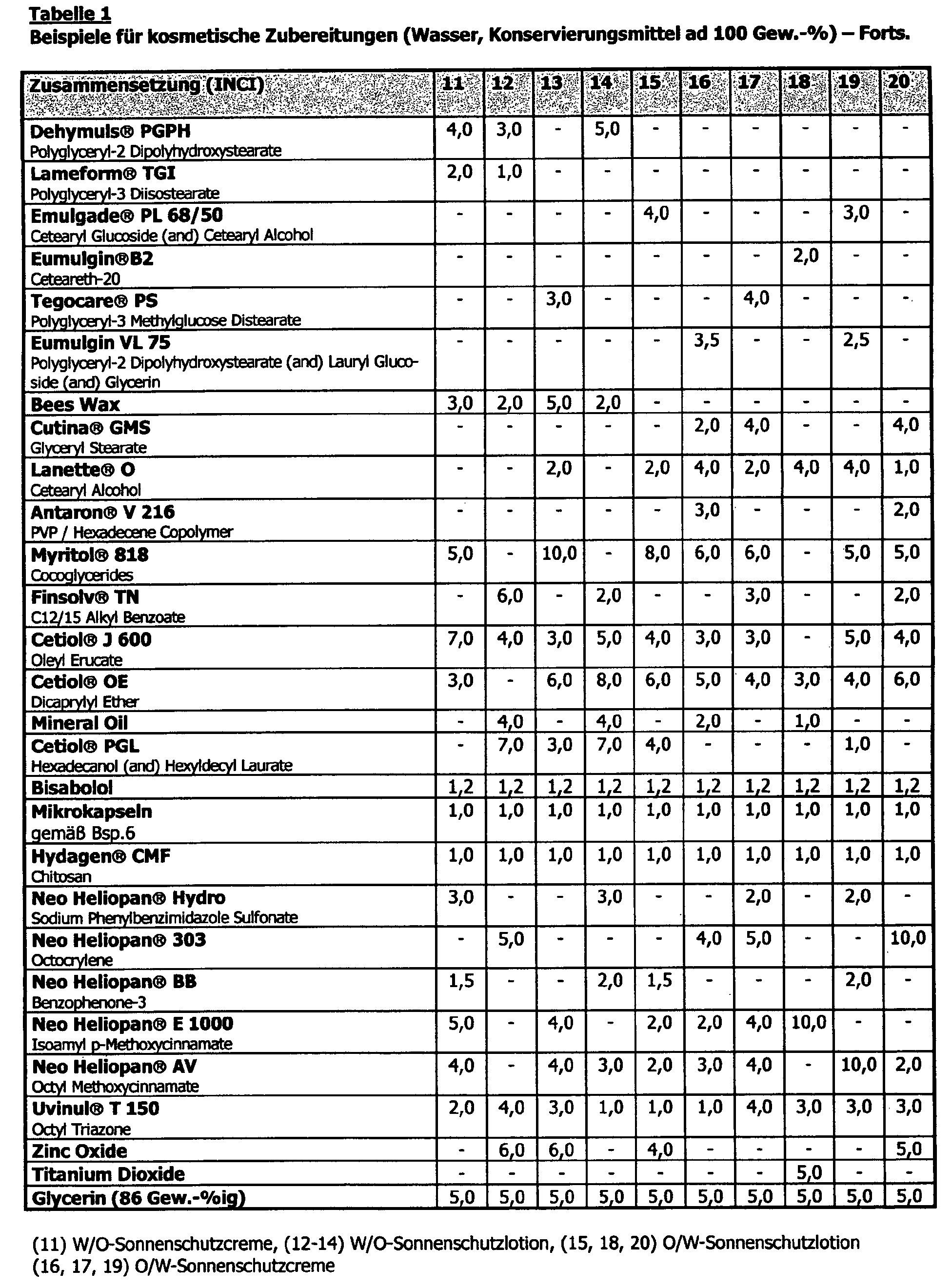

## Patentansprüche

1. Mikrokapseln mit einem mittleren Durchmesser von 100 nm bis 5 mm, bestehend aus einer Hüllmembran und einer davon umschlossenen, die Wirkstoffe enthaltenden Matrix, **dadurch gekennzeichnet, dass** die Matrix eine kubischen Phase darstellt.

2. Verfahren zur Herstellung von Mikrokapseln mit einem mittleren Durchmesser von 100 nm bis 5 mm, bestehend aus einer Hüllmembran und einer davon umschlossenen, die Wirkstoffe enthaltenden Matrix, bei dem man
(a) aus ungesättigten Fettsäurepartialglyceriden und Wasser eine kubische Phase herstellt,
(b) die kubische Phase mit Wirkstoffen und Chitosanen belädt,
(c) die so erhaltene Matrix in Wasser oder einer Ölphase dispergiert, und
(d) die so erhaltene Dispersion mit anionischen Polymeren in Kontakt bringt.

3. Verfahren zur Herstellung von Mikrokapseln mit einem mittleren Durchmesser von 100 nm bis 5 mm, bestehend aus einer Hüllmembran und einer davon umschlossenen, die Wirkstoffe enthaltenden Matrix, bei dem man
(a) aus ungesättigten Fettsäurepartialglyceriden und Wasser eine kubische Phase herstellt,
(b) die kubische Phase mit Wirkstoffen und anionischen Polymeren belädt,
(c) die so erhaltene Matrix in Wasser oder einer Ölphase dispergiert, und
(d) die so erhaltene Dispersion mit einer wässrigen Chitosanlösung in Kontakt bringt.

4. Verfahren zur Herstellung von Mikrokapseln mit einem mittleren Durchmesser von 100 nm bis 5 mm, bestehend aus einer Hüllmembran und einer davon umschlossenen, die Wirkstoffe enthaltenden Matrix, bei dem man
(a) aus ungesättigten Fettsäurepartialglyceriden und Wasser eine kubische Phase herstellt,
(b) die kubische Phase mit Wirkstoffen und einem Alkylendiamin belädt,
(c) die so erhaltene Matrix in einer Ölphase dispergiert, und
(d) die so erhaltene Dispersion mit einem Dicarbonsäurechlorid in Kontakt bringt.

5. Verfahren zur Herstellung von Mikrokapseln mit einem mittleren Durchmesser von 100 nm bis 5 mm, bestehend aus einer Hüllmembran und einer davon umschlossenen, die Wirkstoffe enthaltenden Matrix, bei dem man
(a) aus ungesättigten Fettsäurepartialglyceriden und Wasser eine kubische Phase herstellt,
(b) die kubische Phase mit Wirkstoffen und einem Dicarbonsäurechlorid belädt,
(c) die so erhaltene Matrix in Wasser dispergiert, und
(d) die so erhaltene Dispersion mit einem Alkylendiamin in Kontakt bringt.

6. Verfahren zur Herstellung von Mikrokapseln mit einem mittleren Durchmesser von 100 nm bis 5 mm, bestehend aus einer Hüllmembran und einer davon umschlossenen, die Wirkstoffe enthaltenden Matrix, bei dem man
(a) aus ungesättigten Fettsäurepartialglyceriden und Wasser eine kubische Phase herstellt,
(b) die kubische Phase mit Wirkstoffen und Proteinen belädt, und
(c) die so erhaltene proteinhaltige Matrix durch Temperaturerhöhung denaturiert.

7. Verfahren nach mindestens einem der Ansprüche 2 bis 6, **dadurch gekennzeichnet, dass** man kubischen Phasen herstellt, die ungesättigte Fettsäurepartialglyceride und Wasser im Gewichtsverhältnis 50 : 50 bis 90 : 10 enthalten.

8. Verfahren nach mindestens einem der Ansprüche 2 bis 7, **dadurch gekennzeichnet, dass** man als ungesättigte Fettsäurepartialglyceride Öisäuremono/diglyceride einsetzt.

9. Verfahren nach mindestens einem der Ansprüche 2 bis 8, **dadurch gekennzeichnet, dass** man Wirkstoffe einsetzt, die ausgewählt sind aus der Gruppe der Vitamine, Enzyme, Pflanzenextrakte, Oligosaccharide, Biopolymeren, Proteine, Nucleinsäuren, Bioactiva sowie deren Gemischen.

10. Verfahren nach mindestens einem der Ansprüche 2 bis 9, **dadurch gekennzeichnet, dass** man als Wirkstoffe Flammschutzmittel einsetzt.

11. Verfahren nach mindestens einem der Ansprüche 2 bis 10, **dadurch gekennzeichnet, dass** man die Wirkstoffe - bezogen auf die kubische Phase - in Mengen von 1 bis 25 Gew.-% einsetzt.

12. Verfahren nach den Ansprüchen 2 und/oder 3, **dadurch gekennzeichnet, dass** man Chitosane einsetzt, die ein mittleres Molekulargewicht im Bereich von 10.000 bis 500.000 bzw. 800.000 bis 1.200.000 Dalton aufweisen.

13. Verfahren nach mindestens einem der Ansprüche 2, 3 und/oder 12, **dadurch gekennzeichnet, dass** man anionische Polymere einsetzt, die ausgewählt sind aus der Gruppe, die gebildet wird von Salzen der Alginsäure, anionischen Chitosanderivaten, Poly(meth)acrylaten und Carboxymethylcellulosen.

14. Verfahren nach mindestens einem der Ansprüche 2 bis 4, **dadurch gekennzeichnet, dass** man als Ölphase kosmetische Öle einsetzt, die ausgewählt sind aus der Gruppe, die gebildet wird von Guerbetalkoholen auf Basis von Fettalkoholen mit 6 bis 18 Kohlenstoffatomen, Estern von linearen C₆-C₂₂-Fettsäuren mit linearen C₆-C₂₂-Fettalkoholen, Estern von verzweigten C₆-C₁₃-Carbonsäuren mit linearen C₆-C₂₂-Fettalkoholen, Estern von linearen C₆-C₂₂-Fettsäuren mit verzweigten Alkoholen, Estern von Hydroxycarbonsäuren mit linearen oder verzweigten C₆-C₂₂-Fettalkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈-Fettsäuren, Estern von C₆-C₂₂-Fettalkoholen und/oder Guerbetalkoholen mit aromatischen Carbonsäuren, Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Ölen, verzweigten primären Alkoholen, substituierten Cyclohexanen, linearen und verzweigten C₆-C₂₂-Fettalkoholcarbonaten, Guerbetcarbonaten, Estern der Benzoesäure mit linearen und/oder verzweigten C₆-C₂₂-Alkoholen, linearen oder verzweigten, symmetrischen oder unsymmetrischen Dialkylethern mit 6 bis 22 Kohlenstoffatomen pro Alkylgruppe, Ringöffnungsprodukten von epoxidierten Fettsäureestern mit Polyolen, Siliconölen sowie aliphatischen bzw. naphthenischen Kohlenwasserstoffen.

15. Verfahren nach den Ansprüchen 4 und/oder 5, **dadurch gekennzeichnet, dass** man Alkylendiamine der Formel **(I)** einsetzt,
**H**_{**2**}**N(CH**_{**2**}**)**_{**n**}**NH**_{**2**} (I)
in der n für Zahlen von 4 bis 10 steht.

16. Verfahren nach den Ansprüchen 4, 5 und/oder 15, **dadurch gekennzeichnet, dass** man Dicarbonsäurechloride der Formel **(II)** einsetzt,
**ClOC(CH**_{**2**}**)**_{**m**}**COCl** (II)
in der m für Zahlen von 2 bis 22 steht.

17. Verfahren nach Anspruch 6, **dadurch gekennzeichnet, dass** man als Protein Albumin einsetzt.

18. Verfahren nach den Ansprüchen 6 und/oder 17, **dadurch gekennzeichnet, dass** man die proteinhaltige Matrix bei Temperaturen im Bereich von 35 bis 70 °C denaturiert.

19. Verwendung von Mikrokapseln nach Anspruch 1 zur Herstellung von kosmetischen und/oder pharmazeutischen Zubereitungen.

20. Verwendung von Mikrokapseln nach Anspruch 1 zur Ausrüstung von Fasern und textilen Flächengebilden.
